Europäisches Patentamt

European Patent Office

Office européen des brevets

(19)

(11) Publication number: **0 254 493**
**A1**

# (12) EUROPEAN PATENT APPLICATION

(21) Application number: **87306326.7**

(22) Date of filing: **17.07.87**

(51) Int. Cl.³: **A 61 F 13/02**

(30) Priority: **21.07.86 GB 8617790**

(43) Date of publication of application:
**27.01.88 Bulletin 88/4**

(84) Designated Contracting States:
**AT BE CH DE ES FR GB IT LI NL SE**

(71) Applicant: **Smith & Nephew Associated Companies plc.**
**2 Temple Place Victoria Embankment**
**London WC2R 3BP(GB)**

(72) Inventor: **Grace, Penelope Jane**
**10 Main Street Willerby**
**Hull North Humberside(GB)**

(74) Representative: **Cole, William Gwyn**
**Corporate Patents Department Smith and Nephew**
**Research Limited Gilston Park**
**Harlow Essex CM20 2RQ(GB)**

(54) Wound dressing, manufacture and use.

(57) A non-adherent wound dressing which comprises a central area with at least one handling area (5,6) outwardly disposed from it is described. The central area which is placed over a wound is adhesive-free and is formed from a material which is non-adherent to wounds. The central area comprises a layer of polymer which optionally may be apertured. The handling areas (5,6) may be coated on one surface with a pressure sensitive adhesive which prior to use is covered by a removable protector (7,8). The handling areas (5,6) are used to facilitate application and to adhere the dressing to the body. Optionally an absorbent pad (9) may be in contact with the non-wound contacting surface of the central area to absorb any fluid which may pass through it. In a preferred form the dressing has two adhesive coated handling areas (5,6) on two opposed edges of the central area which central area comprises a film of polymeric material containing a plurality of slits (4).

Fig.1

WOUND DRESSING, MANUFACTURE AND USE

The present invention relates to a wound dressing which consists essentially of an adhesive-free central area which is non-adherent to wounds and which is formed from a layer of synthetic polymer and adhesive coated handling areas outwardly disposed from the central area, the adhesive surfaces of these handling areas being covered by removable protectors. Optionally an absorbent layer is present at the non-wound facing side of the central area. The present invention also relates to the manufacture and use of these dressings.

Wounds such as burns, donor sites, surgical incisions and the like can present a serious problem for their treatment by producing large amounts of exudate which can cause some conventional dressings to become saturated or to adhere to the wound. One approach to solving this problem is to employ materials which are designed to reduce the propensity to adhere to the wound. Such dressings are disclosed in, for example, United States Patents Nos. 2923298 and 3543750 and British Patent No. 778813 and European Patents Nos. 50514 and 122085. However, an unexpected disadvantage arising from the success of such non-adherent dressings is that on application and during use the dressings tend to slip across the surface of the wound thereby disturbing the wound surface or the healing wound.

Conventionally non-adherent dressings are applied by placing the dressing in contact with the wound and then keeping the dressing in place by application of bandages or, in the case of absorbent dressings, lengths of adhesive surgical tape can be used. Usually the lengths of surgical tape are cut from a roll of tape and adhered by one end from a vertical surface until required. The disadvantages of this procedure are that the tape will be exposed to air borne contamination and may adhere to the surface. It has now been found that by providing non-adherent and adhesive-free wound dressings with adhesive coated handling areas the above disadvantages maybe avoided. The dressings have been found to be easier to manipulate during application to the patient because of the rigidity provided by the handling areas. There is less risk of contamination of the wound contacting surface of the dressing by the person applying it as the dressing will be held by the handles. The protector may be removed from one handling area and the dressing anchored to the skin prior to completing the application. The handling areas when all the protectors are removed will serve to hold the dressing in place on the skin.

Accordingly the present invention provides a wound dressing which consists essentially of a central area which is adhesive free and is non-adherent to wounds and which is a layer of polymer, an optional absorbent layer at the non-wound facing side of the central area and handling areas outwardly disposed from said central area which handling areas have pressure sensitive adhesive areas covered by removable protectors.

By 'consists essentially of' when used herein means that the dressing consists of the elements named but does not exclude the presence of any temporary or removable elements which do not form part of the dressing in use or elements which are not necessary for the function of the dressing.

By 'adhesive free' it is meant that there is no adhesive present on the wound contacting surface of the central area, but does not exclude the possibility of the presence of adhesive on the non-wound contacting surface for example for adhering an absorbent layer in contact with the central area.

By layer is meant a film per se or a combination of two or more films.

Aptly the handling areas may be provided at one edge of the central area, two opposite edges of the central area, three edges of the central area or around the periphery of the central area. It is clear therefore that the dressings of the invention will have at least one handling area.

Aptly the handling areas may comprise adhesive coated tape attached to one edge, to two opposite edges, to three edges or around the periphery of the central area. Suitably the handling areas will extend over two opposite side edges of the central area. It is clear therefore that the dressings of the invention will have at least one adhesive coated handling area.

It is clear therefore that the present invention provides a wound dressing which consists essentially of a central area and at least one handling area outwardly disposed from said central area, which central area is adhesive free and is non-adherent to wounds and which comprises a layer of polymer and in which at least one of said handling areas has a pressure sensitive adhesive area covered by a removable protector.

Preferably the wound dressing has two handling areas present at two opposite edges of the central area.

Favourably the adhesive coated tape may be arranged so that part of the tape is attached to the polymer film and the remainder of the tape projects beyond the central area to leave an exposed adhesive coated surface which maybe covered by a removable protector.

The material which may be used to form the handling areas of the wound dressings of the present invention include any of those materials described as suitable for handles in European Patent Application No. 161865 at page 11 line 4 to page 13 line 23, which is incorporated herein by cross-reference.

Particularly preferred materials for forming the handling areas are integral nets particularly those formed by the fibrillation of thermoplastic embossed polyolefin films comprising low and high density polyethylene, polypropylene or copolymers or blends thereof for example blends of polyolefin with polystyrene. Such nets and the polymers used for forming them are described in for example British Patents Nos. 1495151 and 1531715.

Suitable handling areas may also be made from a wide variety of materials including paper, non-woven fabric, woven fabric and films, sheets or webs of polymers including polypropylene, polyethylene, copolymers thereof and blends thereof, polyesters and porous polyvinyl chloride. Preferred handling areas may be made from paper, porous polyvinyl chloride (Porvic, trade mark) or microporous films such as Micropore (trade mark).

Suitably the handling areas may be from 1.0cm to 4.0cm in width and preferably 1.5 to 2.5cm in width, for example 2.0cm, 2.5cm or 3.0cm in width. The width of the margin of the handling areas which is adhered to the edge of the dressing is suitably 0.5 to 1.0cm and preferably is 0.7 to 1.0cm.

Since the handling area is usually to be adhered to the skin, it is apt that the adhesive-coated handling area should have a moisture vapour transmission rate of at least $250 \text{gm}^{-2} \, 24\text{hr}^{-1}$ at 37°C and 100% to 10% relative humidity difference when measured by the Payne Cup Method. More suitably the adhesive coated handling area should have a moisture vapour transmission rate of at least $450gm^{-2}$ $24hr^{-1}$ and preferably should be at least $600gm^{-2}$ $24hr^{-1}$. The handling area may therefore be adhered to the skin, without the risk of causing maceration to the underlying normal healthy skin.

Suitable adhesives for use on the handling areas are described in for example British Patent No. 1280631 and European Patent No. 35399. A particularly suitable adhesive is an acrylate ester copolymer adhesive formed from the polymerisation of 47 parts 2-ethylhexyl acrylate, 47 parts butyl acrylate and 6 parts acrylic acid. This adhesive in combination with a net gives a handling area having a particularly suitable moisture vapour transmission rate. A second suitable adhesive is a polyvinyl alkyl ether adhesive such as polyvinyl ethyl ether adhesive.

The adhesive of the coating on the handling areas may be present as a continuous layer or as a non-continuous layer for example a pattern spread layer, a microporous layer or a porous layer.

Suitably the adhesive layer will be 15 to 65μm thick and will generally have a weight per unit area of 10 to $75gm^{-2}$.

As mentioned hereinabove, the adhesive coated handling areas prior to use will have their exposed adhesive area covered by removable protectors. Suitable protectors include silicone release coated papers, plastics coated papers and release coated films such as silicone coated polyethylene. A favoured release protector is a silicone/polyethylene coated paper known as Steralease (Trade mark).

The protectors may be arranged to cover only the exposed adhesive coating on the handling area or may be adapted to cover also the central area. In one preferred arrangement a single protector covers the central area and two further protectors are present on the handling areas. Another form of three part protector is illustrated in for example European Patent Application No. 117632.

In a second preferred arrangement a protector covers the exposed adhesive coating on one handling area and a second protector is then adapted to cover both the central area of the dressing and the exposed adhesive coating on the second handling area. This may be achieved by folding the second protector longitudinally near to one edge so that the release coated surface can be brought into contact with the exposed adhesive surface of the second handling means as well as the central area of the dressing. In use the first adhesive coated handling area is adhered to the skin, then the second protector may be peeled back until the central area is completely uncovered and the protector is unfolded. The protector is then still attached to the dressing by means of the second handling area. Since the adhesive at this attachment is now under shear force, a slight tension may be applied to the dressing by pulling the protector against the anchorage of the first handling area without the protector separating from the handling area. This tension results in a dressing which contains slits, in the slits opening slightly which results in a better performance from the dressing. The second protector may be removed by peeling from the handling area and the handling area may then be adhered to the skin. Alternatively it may be the second handling area which is folded longitudinally around the edge of the dressing and attached to the non-dressing contacting side of the second protector. The protector is therefore preferably formed from a material which has a release coat on both surfaces. The operation of the dressing is similar to that above.

In a third preferred arrangement the protector is in the form of a split protector in which the two halves overlap in the central area of the dressing and which cover the central area of the dressing and the exposed adhesive surfaces of the handling areas. Suitably each protector will have a longitudinal fold as described above or the handling area will have a longitudinal fold.

In another aspect the present invention provides a wound dressing which consists essentially of a central area which is adhesive free and is non-adherent to wounds and which is a layer of polymer, an optional absorbent layer at the non-wound facing side of the central area and handling areas outwardly disposed from said central area which handling areas have pressure sensitive adhesive areas covered by removable protectors and in which at least one of said protectors is adapted to cover the central area of the dressing as well as the adhesive coating on the handling area.

Preferably the protector is in the form of a split protector whereby each protector on the handling means is adapted to cover a part of the central area of the dressing and the adhesive coating of the handling area. The protectors overlap within the central area of the dressing.

If an absorbent layer is absent in the dressing of the invention then a support frame such as that described in European Patent Application No. 51935 may be present on the non-wound contacting surface of the central area.

The central area of the dressings of the present invention comprises a synthetic polymer layer. In one preferred form the central area will be a film.

The central area may be a continuous film such as a moisture vapour permeable film including those formed from polyurethane, polyvinyl alcohol, cellulose derivatives, elastomeric polyester such as Hytrel (Trade Mark), polyether polyamide such as Pebax (Trade Mark) and the like. Suitably the film may have a moisture vapour transmission rate of greater than $250gm^{-2}$ $24hr^{-1}$ at 37°C and 100% to 10% relative humidity difference and more suitably greater than $500gm^{-2}$ $24hr^{-1}$, most suitably greater than $1000gm^{-2}$ $24hr^{-1}$ and preferably greater than $1800gm^{-2}$ $24hr^{-1}$.

In one preferred form the central area comprises a continuous film of a hydrophilic polymer and in particular of a hydrophilic polyurethane. Suitable polyurethanes may contain when hydrated from 5 to 95% by weight of water, more suitably 15 to 60% by weight of water and preferably from 20 to 40% by weight of water for example 25%, 30% or 40%. Preferred hydrophilic polyurethanes are described in United Kingdom Patent No. 2093190B, expecially in Example 2.

However in a second preferred form the central area may be an apertured film. It has been found to be particularly advantageous to use outwardly disposed handling areas in dressings which employ an adhesive-free apertured film wound contact layer. Non-adherent dressings in which apertured polymer films are used as the wound contact layer may suffer from the disadvantage that they may slide over the wound during and after application thereby disturbing the wound or more diliteriously the healing wound. By using adhesive coated handling areas the dressing may be anchored at one end during application and be securely fastened to the patient after application. Aptly the apertured film used in the present invention may be any of those which have been used as a non-adherent wound contact layer heretofore, for example films formed from polyethylene, polyfluorocarbons and the like. However, preferred materials for forming the apertured wound contact layer include those described in European Patent Application No. 122085 at page 6 line 3 to page 13 line 11, which are incorporated herein by cross-reference. Suitably therefore the wound contact layer comprises a conformable film with apertures in the form of slits therethrough in which the film comprises a first layer for contacting the wound laminated to a second layer, said first layer comprising a material which when in contact with water swells and said second layer comprises a material which when in contact with water does not swell or swells less than the first layer. Thus when in contact with a wet wound the slits open allowing water to pass through the film and when in contact with a dry surface the slits are closed.

A preferred apertured film wound contact layer is formed from a laminate of a hydrophilic polyurethane, whose preparation is described in British Patent No. 2093190B and which will contain from 20 to 40% by weight of water when hydrated and a styrene-butadiene-triblock copolymer. Aptly this laminate may be apertured by cutting slits through the laminate. Normally the slits will be arranged in rows and often in rows in which the slits of any one row will be staggered from those in the next row. Suitably the slits will be from 1 to 15mm long and more suitably 2 to 10mm long. The slits in any row may be suitably from 2 to 10mm apart and the distance between the slits in adjacent rows may suitably be 1 to 15mm, more suitably 3 to 10mm and preferably 4 to 8mm.

A second preferred central area wound contacting layer comprises a perforated polyester film. Suitable perforated polyester films are described in United States Patents Nos. 2923298, 3012659 and 3012918.

An apt central area wound contacting layer comprises a net material. Preferred nets are described in United Kingdom Patent No. 1548865 which is incorporated by cross-reference. A particularly preferred net is described in Example 2 of that patent.

A second suitable net may be formed from an elastomeric polymeric material such as polyurethane or a blend of ethylene-vinyl acetate and polyolefin such as polystyrene. Such nets are described in for example European Patents Nos. 50514 and 141592.

Another apt form of the central area is an open knit fabric whose construction allows exudate to pass freely from the wound but does not adhere the wound. The fabric may be made from rayon filaments.

The wound dressings of the present invention may or may not include an absorbent layer at the non-wound facing side of the central area. If an absorbent layer is not present then it is conventional to place an absorbent layer in contact with the central area of the dressing after the dressing has been positioned on the patient. The added absorbent layer may be then maintained in contact with the central area of the dressing by conventional bandaging means. It is an advantage of this type of dressing of the present invention that when the handling areas have been used to adhere the wound contacting central area to the skin then, if necessary, a soiled absorbent layer may be removed and replaced by a fresh absorbent layer without disturbing the wound. Suitably the absorbent layer may be formed from materials including gauze, cellulose pads, synthetic polymer foam and other conventional body fluid absorbing materials.

When an absorbent is present in the dressings of the invention it may be any of those which are conventionally used in absorbent dressings and may include gauze or cellulose pads or a synthetic polymer foam particularly when formed from a hydrophilic polymer. A preferred absorbent comprises a cotton/acrylic fibre fleece. Other absorbents include mixtures of cotton and viscose, acrylic fibres and the like. A second preferred absorbent comprises a hydrophilic polyurethane foam, for example a Hypol hydrophilic polyurethane foam as described in European Patents Nos. 59048, 59049, 97517 and 106439 which are incorporated herein by cross-reference.

Suitably the absorbent layer will have a width which is approximately equal to the central area that is the distance between the handling areas, however it is not disadvantageous if the absorbent layer overlaps slightly onto the handling areas. Of course if the central area comprises an apertured film the absorbent layer aptly covers at least the area which is apertured so as to avoid the risk of unsightly leakage of body fluid.

The dressings of the present invention may be prepared by forming a film of the synthetic polymer which is to form the central area, for example by extrusion or by casting from a solution of the polymer. The film may then be apertured, if required, by conventional means for example flame perforation, cutting slits, embossing and fibrillation and the like. The adhesive coated handling area is then attached to the central area at the appropriate positions, the remaining exposed adhesive surface of the handling area being covered by protectors. If present, the absorbent layer, pre cut to the appropriate width is laminated to the central area between the handling areas by conventional means, for example spot welding or by the application of heat and pressure or by adhesive. The resulting strip of dressing material may be cut transversely to provide dressings of the desired length. Suitably the dressings may be from 5 x 5cm to 20 x 20cm in size.

The wound dressing of the invention is preferably sterile. The wound dressing is therefore advantageously provided within a bacteria proof pack such as a sealed aluminium foil or paper/plastic film pouch. Sterilization of the dressing can be carried out by a conventional sterilizing method such as ethylene oxide, electron or gamma irradiation.

In use the wound dressing is removed from the pack and any protector present on the central area removed, then a protector covering the adhesive coating on one handling area may be removed and the handling area adhered to the patient's skin thereby providing an anchor point for the dressing while the remainder of the dressing is manoeuvred into position. The protector on the other handling area may then be removed and the handling area adhered to the skin.

In a second embodiment of the present invention in which an absorbent means is present in the wound dressing the handling areas may be attached to the absorbent material rather than to the polymer film.

In a third embodiment of the present invention the central area may comprise a non-adherent wound dressing as described in European Patents Nos. 59048 and 59049 and which comprise an elastomeric net wound contact layer, an intermediate hydrophilic foam absorbent and optionally a moisture vapour permeable film outer layer. Handling areas may be attached to the wound dressings described therein in the manner described hereinbefore.

In a further aspect the present invention provides a method of forming a wound dressing consisting essentially of a central area of a non-wound adherent and adhesive free layer of polymer, an optional absorbent layer and adhesive coated handling areas disposed outwardly from the central area in which the adhesive is covered by a removable protector which method comprises forming the central area, attaching the handling areas to the central area covering the exposed pressure sensitive adhesive areas on the handling areas with a removable protector and when present attaching the absorbent layer to the non-wound contacting face of the central area.

Optionally the central area may contain medicament. Suitable medicaments include broad spectrum antibacterial agents which may be released to the surface to which the dressing is applied. Suitable antibacterial agents include silver sulphadiazine, chlorhexidine salts and salts of polymeric biguanides, known as Vantocils (Trade Marks). Favoured antibacterial agents include salts of chlorhexidine such as its dihydrochloride, diacetate and digluconate.

Other suitable medicaments include antiflammatories, analgesics, local anaesthetics and antifungal agents such as the azole-type compounds.

Signified by miconazole, clotrimazole and metronidazole and combinations of antibacterial agents such as azole-type compounds with silver compounds, for example silver sulphadiazine and metronidazole.

Suitably the central area will contain an effective amount of the medicament and for example may contain upto 50% by weight of the central area of antibacterial agent, more suitably from 5 to 35% by weight and preferably 10 to 25% by weight of antibacterial agent.

Medicament may be present in the absorbent layer and the adhesive layer of the handling areas. Suitably the medicament may be of the same type as may be present in the central area and in the same proportions.

In a final aspect the present invention provides a method of treating a wound by removing a protector from a handling area of a dressing of the present invention and adhering the handling area to the skin, placing the central area of the dressing over the wound and removing the protectors from other handling areas before adhering said areas to the skin.

Preferred embodiments of the present invention will be described by way of example with reference to the accompanying drawings in which

Figure 1 represents a cross-section through a dressing of the present invention in which the central area is covered by a protector

Figure 2 shows a cross-section through a dressing with adhesive coated handling areas in which a portion of their adhesive surface is covered by protectors.

Figure 3 shows a cross-section through a dressing in which the protector is folded at one end and attached to a handling area.

Figure 4 shows a cross-section through a dressing in which the protector is a split protector.

Figure 5 shows a cross-section through a dressing in which one of the handling areas is folded.

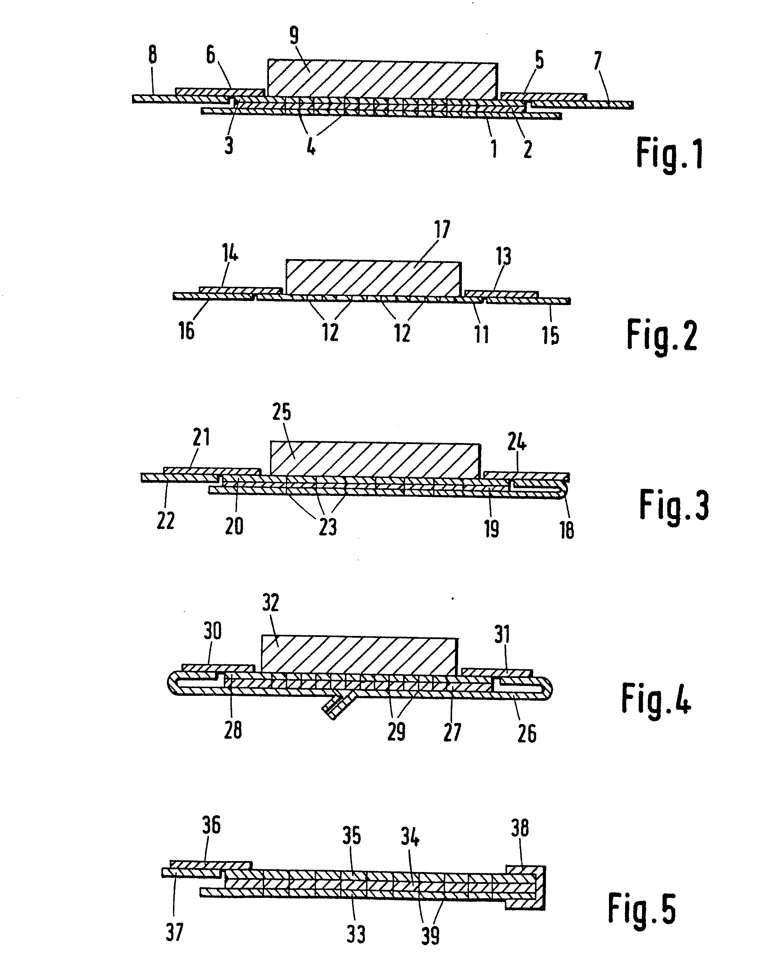

Figure 1 shows a cross-section through a dressing of the present invention in which the release sheet (1) is a sheet onto which a water swellable film (2) is cast. A non-water swellable film (3) is laminated to the film (2) usually by passing the two films and the release paper between a pair of heated rollers under pressure. Slits (4) are then placed through the laminate and the release sheet. The handling areas are represented by adhesive coated films (5, 6). About one-third of their width is used to adhere the handling areas to the non-water swellable film (3). The remainder of the adhesive surface of the handling areas is covered by protectors (7, 8). An absorbent material (9) is arranged to have a width so that it fits between the adhesive coated film (5, 6) but there may be some overlap of the absorbent onto the handling areas. Usually the handling areas will be identifiable by being pigmented.

In use the release paper (1) is removed from covering the central area and then one of the protectors (7) is removed. The adhesive on the handling area so revealed may be used to adhere one edge of the dressing to the skin to serve as an anchor point as the central area of the dressing is placed over the wound. Then the remaining protector (8) is removed and the other handling area is adhered to the skin and the dressing is in place.

Figure 2 shows a cross-section through a dressing of the present invention comprising a perforated polyester film (11) having a series of perforations (12) across its width. Two opposite edges of the polyester film are attached to handling areas (13, 14) comprising adhesive coated strips of a thermoplastic fibrillated net material. The strips of net are attached so that a major portion of the adhesive surface is not adhered to the polyester film but is instead covered by a siliconised release paper protector (15, 16). An absorbent cotton/acrylic fibre fleece (17) is positioned between the handles and is attached to the polyester film by spot welding.

In use one of the protectors (15) may be removed from the adhesive surface of the handling area and adhered to the skin. This may serve to anchor the dressing as the absorbent portion is positioned over the wound. The second protector may then be removed and the handling area adhered to the skin. In this way the dressing is conveniently adhered to the skin of the patient.

Figure 3 shows a cross-section through a dressing of the present invention in which a protector (18) is adapted to cover the central area of the dressing and the exposed surface of a handling area (24). The protector is folded near to one end so that the siliconised surface contacts the exposed adhesive coating of the handling area (24). A water swellable film (19) is laminated to a non-water swellable film (20). The combination of the protector (18), and films (19,20) are apertured by means of slits (23) over the central area of the dressing. Protector (18) is adapted to cover the exposed surface of a handling area (24) along one edge of the dressing, the opposite edge of the dressing has a handling area (21) comprising an adhesive tape and a removable protector (22). An absorbent material (25) may be arranged between handling areas (21) and (24).

In use the adhesive coating on the handling area (21) is exposed by removing the protector (22). The handling area (21) is then adhered to the skin. The protector (18) is then peeled back from the central area until the central area is uncovered and the fold in the protector (18) is unfolded. The tension of unpeeling this protector (18) causes the slits (23) in the dressing to open slightly. The protector (18) is then removed from handling area (24) and the handling area (24) adhered to the skin.

Figure 4 shows a cross-section through a dressing of the present invention in which the protector (26) is in the form of a split protector the two halves of which overlap in the central region of the dressing and completely cover one surface of a water swellable film (27). Each of the halves of the split protector (26) is folded near its edge so that the release surface contacts the exposed adhesive coating of the handling areas (30) and (31). A water swellable film (27) is laminated to a non-water swellable film (28). The laminate contains slits (29). An absorbent material (32) may be arranged between the handling areas (30) and (31).

In use the split protector is peeled back to expose the dressing. When the protectors are fully extended the adhesive on the handling area is under shear so the protectors do not seperate from the adhesive surface. The dressing may be placed over the wound and the parts of the protector removed by peeling from the adhesive coating on the handling area and the handling areas adhered to the skin.

Figure 5 shows an alternative form of protector arrangement whereby instead of the protector being folded, the handling area (38) is folded longitudinally around the edge of the laminate films (34,35) and is attached to the surface of the protector (33) which is not in contact with the laminate films (34,35). The opposed edge of the laminate film carries a handling area (36) whose exposed adhesive surface is covered by a protector (37). In use the protector (37) is removed and the handling area (36) adhered to the skin.  The protector (33) is peeled away from the surface of the film (34) until the surface is completely uncovered. The protector (33) being attached to film (35) by the handling means (38) can be used to apply the bandage under tension so that slits (39) are slightly opened. The protector (33) may be peeled from handling area (38) and the area adhered to the skin.  An absorbent pad may be placed in contact with the film (35) if necessary. It is preferred in the embodiment that the protector (33) is covered on both major surfaces by a release coating.

Example 1

A laminate comprising a silicone release paper, a hydrophilic polyurethane and a styrene-butadiene-styrene tri-block copolymer was prepared in a similar manner to that described in Example 1 of European Patent Application No. 122085.

The films and release paper were then perforated by slits 2mm in length spaced 2mm apart with 5mm spacing between lines of adjacent slits.

An adhesive coated fibrillated thermoplastic film material formed by the method described in British Patent No. 1531715 was applied to two opposed edges of the perforated film to form handling areas. The adhesive surface of the portion of each handling area not adhered to the perforated film was covered by a silicone release paper.

An absorbent material comprising a cotton/acrylic fleece was cut to the width of film exposed between the handling areas and the fleece laminated to the perforated film using a Reliant laminator at a temperature of 100°C and pressure of 20 psi.

The dressing is cut to the appropriate length and packaged in a bacteria proof pack which may be sealed and sterilised by gamma irradiation or ethylene oxide.

Example 2

A dressing is prepared in a similar manner to that described in Example 1 except that the absorbent material used is a hydrophilic polyurethane foam.

Example 3

A dressing is prepared in a similar manner to that described in Example 1 except that the adhesive coated handles were attached to the absorbent material.

Example 4

A dressing is prepared by forming a laminate comprising a silicone release paper, a hydrophilic polyurethane and a styrene-butadiene-styrene-triblock copolymer employing a similar method to that described in Example 1 of European Patent Application No. 122085.

The films on the release paper were then perforated by slits 3mm in length spaced 2mm apart and with 7mm spacing between lines of adjacent slits.

An adhesive coated fibrillated thermoplastic net material formed by the method described in British Patent No. 1531715 is applied to two opposed edges of the perforated film to form handling areas. The adhesive surface of the portion of each handling area not adhered to the perforated film is covered by a silicone release paper.

The dressing is then cut to the appropriate length and packaged in a bacteria proof pack which is sealed and sterilised by gamma irradiation or by ethylene oxide.

In use the dressing is removed from the bacteria proof pack. The silicone release paper is removed from the apertured central area. A protector is then removed from a handling area which is adhered to the skin. The remainder of the dressing is arranged over the wound and the second protector removed and the second handling area adhered to the skin. A separate absorbent layer may then be placed over the central area and held in place by a conventional bandage for example a linear or tubular elastic bandage.

## Example 5

A flame-perforated polyester film is slit to a width of 8.5cm. An adhesive-coated tape formed from a fibrillated thermoplastic net comprising a blend of polyethylene and polystyrene coated with an acrylic ester copolymer pressure sensitive adhesive and 2.5cm wide is applied to two opposite edges of the polyester film so that approximately 0.5mm of the strip is adhered to the edge of the polyester film. The remainder of the adhesive coated surface of the tape is covered by a release paper. A strip of cotton/acrylic fibre fleece, 7,5cm wide, is placed between the handles and spot welded to the exposed polyester film surface. Dressings of the required size are formed by cutting transversely across the strip so formed.

The dressings are packaged in a bacteria proof pack and sterilised by ethylene oxide or gamma irradiation.

In use the sterile dressings may be removed from the pack and the protector paper removed from one of the adhesive coated handles. The handle may then adher to the skin while the remainder of the dressing is positioned over the wound. The second protector may then be removed and the second handle adhered to the skin.

Example 6

A dressing is prepared by taking a net formed in the manner described in Example 2 of British Patent No. 1548865 and applying to two opposed edges of the net an adhesive coated fibrillated thermoplastic net material formed by the method described in British Patent No. 1531715. The adhesive surface of the portion of each handling area not adhered to the net is covered by a silicone coated release paper.

The dressing may be packaged in a bacteria proof pack and sterilised as described herein before.

Example 7

A dressing is prepared by taking an open knit rayon fabric and applying to two opposed edges of the fabric an adhesive coated fibrillated thermoplastic net material formed by the method described in British Patent No. 1531715. The adhesive surface of the portion of each handling area not adhered to the fabric is covered by a silicone coated release paper.

## CLAIMS

1.    A wound dressing which consists essentially of a central area and at least one handling area outwardly disposed from said central area, which central area is adhesive free and is non-adherent to wounds and which comprises a layer of polymer and in which at least one of said handling areas has a pressure sensitive adhesive area covered by a removable protector.

2.    A wound dressing as claimed in claim 1 in which the handling areas are provided at two opposite edges of the central area.

3.    A wound dressing as claimed in either of claims 1 or 2 in which the handling area is net formed by the fibrillation of thermoplastic embossed polyolefin film.

4.    A wound dressing as claimed in any one of claims 1 to 3 in which the adhesive-coated handling area has a moisture vapour transmission rate of at least $250 \text{gm}^{-2}$ $24 \text{hr}^{-1}$ at $37\,^{\circ}C$ and 100% to 10% relative humidity difference.

5.    A wound dressing as claimed in any one of claims 1 to 4 in which all of each handling area is coated with a pressure sensitive adhesive.

6. A wound dressing as claimed in any one of claims 1 to 5 in which the central area is an apertured film.

7. A wound dressing as claimed in claim 6 in which the central area is a conformable film with apertures in the form of slits there through said conformable film comprising a first layer for contacting the wound laminated to a second layer, said first layer comprising a material which when in contact with water swells and said second layer comprising a material which when in contact with water does not swell or swells less than the first layer.

8. A wound dressing as claimed in claim 7 in which the first layer is a hydrophilic polyurethane and the second layer is styrene-butadiene triblock copolymer.

9. A wound dressing as claimed in claim 6 in which the apertured film is a perforated polyester film.

10. A wound dressing as claimed in claim 6 in which the apertured film is a net.

11. A wound dressing as claimed in claim 6 in which the central area is an open knit rayon fabric.

12. A wound dressing as claimed in claim 1 in which the central area is a continuous, moisture vapour permeable film, which film has a moisture vapour transmission rate of greater than 250 gm$^{-2}$ 24 hr$^{-1}$ at 37°C and 100% to 10% relative humidity difference.

13. A wound dressing as claimed in claim 12 in which the film comprises a hydrophilic polyurethane which contains from 20 to 40% by weight of water when hydrated.

14. A wound dressing as claimed in any one of claims 1 to 13 in which an absorbent layer is additionally present at the non-wound facing side of the central area.

15. A wound dressing as claimed in claim 14 in which the absorbent layer is a cotton/acrylic fibre fleece.

16. A wound dressing as claimed in claim 14 in which the absorbent layer is a hydrophilic polyurethane foam.

17. A wound dressing as claimed in any one of claims 1 to 16 in which the pressure sensitive adhesive is an acrylate ester copolymer adhesive and is present at a weight per unit area of 10 to 75gm$^{-2}$.

18.    A wound dressing as claimed in any one of claims 1 to 17 in which the central area contains a medicament.

19.    A wound dressing as claimed in claim 18 in which the medicament is antibacterial and comprises from 1 to 25% by weight of the central area.

20.    A wound dressing as claimed in any one of claims 1 to 19 which is sterile and is packaged in a bacteria-proof pack.

Fig.1

Fig.2

Fig.3

Fig.4

Fig.5

**European Patent Office**

## EUROPEAN SEARCH REPORT

Application number

EP 87 30 6326

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,X | EP-A-0 122 085 (SMITH & NEPHEW) * Page 13, lines 12-22; page 21, lines 1-12; page 11, lines 3-14; page 13, lines 11-14 * | 1 | A 61 F 13/02 |
| Y | | 2-20 | |
| D,Y | EP-A-0 161 865 (SMITH & NEPHEW) * Whole document * | 2-9,12 -20 | |
| D,Y | GB-A-1 548 865 (SMITH & NEPHEW) * Page 1, lines 10-13; claim 1 * | 10 | |
| Y | US-A-4 561 435 (McKNIGHT) * Column 2, lines 59-66; figures * | 11 | TECHNICAL FIELDS SEARCHED (Int Cl 4) |
| A | FR-A-2 531 627 (HOLDING EUROPEENNE DE TEXTILE) | | A 61 F |
| A | US-A-3 903 882 (AMERICAN CYANAMID CO.) | | |

The present search report has been drawn up for all claims

| Place of search | Date of completion of the search | Examiner |
|---|---|---|
| THE HAGUE | 26-10-1987 | STEENBAKKER J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82